Europäisches Patentamt

European Patent Office

Office européen des brevets

(19)

(11) Publication number: **0 146 320**
**A2**

# (12) EUROPEAN PATENT APPLICATION

(21) Application number: **84308524.2**

(22) Date of filing: **07.12.84**

(51) Int. Cl.⁴: **A 61 F 13/20**

(30) Priority: **21.12.83 GB 8334089**

(43) Date of publication of application: **26.06.85**
**Bulletin 85/26**

(84) Designated Contracting States: **AT BE DE FR GB SE**

(71) Applicant: **Smith and Nephew Associated Companies p.l.c., 2, Temple Place Victoria Embankment, London WC2R 3BP (GB)**

(72) Inventor: **Reynolds, Nigel Charles, 12 Hitch Common Road Newport, Saffron Walden Essex (GB)**

(74) Representative: **Cole, William Gwyn, Corporate Patents Department Smith and Nephew Research Limited Gilston Park, Harlow Essex CM20 2RQ (GB)**

(54) Tampon insertion device.

(57) A tampon device for insertion into a body cavity which device comprises a tampon and a flexible tube, adapted to allow transitional movement of the tampon therein, which tube has a collapsed portion at or about the nose of the tampon and portion adjacent to the collapsed portion which covers at least a part of the tampon rearward of the nose thereof and which portion is capable of extending from the collapsed portion to form a sleeve about the tampon when the device is being inserted into a body cavity, tampon assemblies and tampon applicators comprising such a device are described.

1

# TAMPON INSERTION DEVICE

The present invention relates to tampon devices, tampon assemblies and applicators for aiding the insertion of a tampon into a body cavity.

Conventional tampons can be relatively uncomfortable to insert into or withdraw from a body cavity such as a vagina due to friction between the walls of the body cavity and the surface of the tampon. United States Patent No. 4,211,225 discloses a tampon suitable for insertion into a vagina which has a collapsible water permeable shroud affixed to the front end of the tampon. The shroud has an inner layer which reduces the sliding friction between the tampon and the shroud and allows the tampon to slide from the shroud on withdrawal of the tampon from the vagina. The shroud during withdrawal collapses and inverts in a rolling action against the walls of the vagina thereby making withdrawal of the tampon relatively comfortable. During insertion of the tampon however, the shroud is in sliding contact with the walls of the vagina which may make the insertion of the tampon relatively uncomfortable. A tampon device has now been found which substantially reduces frictional contact between the device and the walls of the body cavity during its insertion therein.

Accordingly the present invention provides a tampon device for insertion into a body cavity which device comprises a tampon and a flexible tube, adapted to allow transitional movement of the tampon therein, which tube has a collapsed portion at or about the nose of the tampon and a portion adjacent to the collapsed portion which covers at least a part of the tampon rearward of the nose thereof and which portion is capable of extending from the collapsed portion to form a sleeve about the tampon when the device is being inserted into a body cavity.

The portion of the flexible tube of the tampon device of the invention which is adjacent to collapsed portion and which covers part of the tampon rearward of the nose thereof, that is the cover portion of the tube, does not enclose the collapsed portion. A tampon device in which the collapsed portion is enclosed within the flexible tube so that the collapsed portion everts during the insertion of the tampon into the body cavity is the subject of European Patent Application No. 83305319.2, published as No. 0104039.

The collapsed portion of the tube of the tampon device of the invention will usually be in a folded configuration. On insertion of the tampon device into a body cavity, contact between the body cavity and the cover portion of the tube causes the collapsed portion to unfold and the cover portion to extend therefrom and form a sleeve about the moving tampon. The unfolding action of the tube allows the sleeve to be layed down against the walls of the body cavity in a substantially non-frictional manner thereby reducing any discomfort which may be caused by frictional contact of the tampon device therewith. Furthermore, the sleeve prevents or reduces contact of the surface of the moving tampon with the walls of the body cavity.

The tampon device of the invention can be inserted into a body cavity by digital pressure on the rear end of the tampon.

The tampon device of the invention can be provided with a guide to aid insertion of the tampon.

Accordingly in another aspect the invention provides a tampon assembly for aiding the insertion of a tampon into a body cavity comprising a tampon device which comprises a tampon and a flexible tube, adapted to allow transitional movement of the tampon therein, which tube

4

has a collapsed portion at or about the nose of the tampon and a portion adjacent to the collapsed portion which covers at least a part of the tampon rearward of the nose thereof and which portion is capable of extending from the collapsed portion to form a sleeve about the moving tampon when the device is being inserted into a body cavity and a tampon guide for receiving the tampon having an opening to be placed over the body cavity opening.

Insertion of the tampon into the body cavity using the tampon assembly can be achieved by digital pressure on the rear end of the tampon.

The cover portion of the flexible tube used in the tampon assembly of the invention can be attached to the tampon guide for example around its opening. Such an attachment can assist in extending the cover portion of the flexible tube when the tampon is inserted into the body cavity. It is preferred, however, that the attachment is frangible or water soluble so that the tampon guide can be discarded after the tampon has been inserted.

The tampon device of the invention can be part of an applicator for aiding insertion of the tampon. Accordingly in a further aspect the invention provides an

5

applicator for aiding the insertion of a tampon into a
body cavity comprising (1) a tampon device which comprises
a tampon and a flexible tube, adapted to allow
transitional movement of the tampon therein, which tube
has a collapsed portion at or about the nose of the tampon
and a portion adjacent to the collapsed portion which
covers at least a part of the tampon rearward of the nose
thereof and which portion is capable of extending from the
collapsed portion to form a sleeve about the moving tampon
when the device is being inserted into the body cavity;
(2) an applicator tube containing the tampon device and
(3) a piston capable of sweeping the applicator tube and
ejecting the tampon device therefrom.

The piston of the applicator of the invention is
preferably in the form of a tube which can slide inside
the applicator tube in a manner similar to that of a
telescope. The cover portion of the flexible tube can be
attached to the front end of the applicator tube. Such an
attachment can assist in extending the cover portion of
the flexible tube when the tampon is inserted into the
body cavity. The attachment however, should be frangible
or water soluble so that the applicator tube and piston
can be discarded after use. In a preferred applicator of
the invention, however, the cover layer of the flexible

6

tube is not attached to the applicator tube.

The applicator of the invention may be used in a conventional manner to insert a tampon or tampon device of the invention by inserting the applicator tube into the body cavity and ejecting the tampon therefrom by means of the piston.

Suitable applicator tube, piston and tampon guide components for use in the invention can be made of a biocompatible plastics or coated paper materials which are conventionally used for such components.

The tampon used in the tampon device, tampon assembly and applicator of the invention can be any of the tampons conventionally used for medical or sanitary purposes for example a vaginal tampon. Conventional tampons normally comprise absorbent compressed fibres such as viscose or cotton fibres or absorbent compressed foam. Such tampons are capable of expanding when contacted with aqueous fluids to block the body cavity into which they have been inserted. Suitable tampons for use in the invention have an approximately cylindrical shape. The tampon may have circumferential crimping lines to aid the expansion and absorption of the tampon. The tampon will usually have a rounded nose portion to aid the insertion of the tampon.

7

Typical vaginal tampon can have a length of 4 to 6 cm and a maximum diameter of approximately 4 to 20 mm to enable the tampon to be inserted into a normal vaginal tract.

The tampon used in the invention can have a nose portion which has a smaller diameter than that of the main body of the tampon to accomodate a collapsed portion of the flexible tube positioned about the nose of the tampon. The collapsed portion can favourably be an axially compressed portion as described hereinafter.

The tampon used in the invention can advantageously have a cover layer of a liquid permeable material. The liquid permeable material of the cover layer can conveniently be the material used for the flexible tube used in the invention as will be described hereinafter. The cover layer may be over substantially the whole surface of the tampon or in the form of a shroud which surrounds the tampon. The cover layer over the tampon can reduce the friction between the surface of the tampon and the flexible tube during insertion of the tampon into the body cavity. In order to further reduce the friction between cover layer on the tampon and the flexible tube either of these layers can be provided with a coating of a pharmaceutically acceptable lubricant or release agent

8

which includes silicone oils and emulsions, silicone resins and fluoroethylene polymers such as P.T.F.E. and the like. The cover layer of the tampon if desired can be attached to the surface of the tampon for example by adhesive. A shroud cover layer will usually be attached at or about the nose of the tampon. Such attachment can be achieved by any suitable adhesive or mechanical method.

The tampon used in the invention can optionally contain a medicament or a topically anti-infective agent.

The tampon used in the invention will normally be provided with a conventional withdrawal cord or string attached to the rear end of the tampon. The flexible tube used in the invention may be adapted to provide an alternative withdrawal means.

The flexible tube used in the invention can include circumferential continuous tubes and tubes in the form of rolled sheet. Such rolled sheets can overlap or be joined at its long edges for example by adhesive or a suitable sealing method such as heat sealing. Favourably the flexible tube is adapted to allow the tampon to slide in a low frictional manner through the tube. The tube therefore will usually have diameter greater than the maximum girth of the tampon. Suitable tubes for use with a vaginal tampon will have diameter of at least 10 mm and preferably a diameter of at least 20 mm. The flexible tube should also be capable of accomodating the expansion of the tampon in use. The tube therefore may be capable of circumferential expansion for by example the provision of axial pleats or slits in the length of the tube.

The flexible tube used in the invention extends during insertion of the tampon or tampon device of the invention into a body cavity to form a lining on the wall of the body cavity. The flexible tube will therefore be made of a biocompatible material. The length of the flexible tube can be adapted to the size of the body cavity into which the tampon is to be inserted. The length of flexible tube for lining an adult vagina can suitable be 30 mm to 200 mm and preferably 140 mm to 175 mm.

The forward part of the extended flexible tube surrounds the tampon after the tampon device has been inserted into the body cavity. At least the forward part of the flexible tube will therefore be liquid permeable to allow passage of body exudate through the tube to the tampon. A flexible tube suitable for a tampon device for insertion into an adults vagina will usually have a liquid

10

permeable forward part of about 4 to 6 cm long. It is preferred however, that the whole of the flexible tube is made of a liquid permeable material.

Suitable flexible liquid permeable tube structures include nets, non-woven fabrics, woven or knitted fabrics and porous films. Apt structures comprise flexible thin or light weight net, non-woven fabric or perforated film materials.

Suitable nets and non-woven fabrics have a thickness of 10 micro metres to 400 micro metres and preferably a thickness of 75 micro metres to 150 micro metres.

Suitable nets and non-woven fabrics have a weight per unit area of 5 to 160 $g/m^2$ more suitably a weight per unit area of 10 to 160 $g/m^2$ and preferably a weight per unit area of 10 to 60 $g/m^2$.

Suitable nets and non-woven fabrics comprise a plastics material which includes polyamides, polyolefins such as polyethylene and polyurethane.

Apt non-woven fabric materials for the flexible tubes used in the invention include spun-bonded polyamide (weight per unit area 16 $g/m^2$) known as "cerex" available from Monsanto, and bonded polypropylene (weight

11

per unit area 20 g/m$^2$ and thickness of 50 micro metres) known as "Corovin SMED" available from J.H. Benecke GmbH.

Apt net materials for flexible tubes used in the invention are high density polyethylene nets made according to British Patent No. 1,548,865 known as Net 909 Grade A4 available from Smith & Nephew Plastics Limited.

Suitable perforate films for the flexible tubes used in the invention will be made of a flexible water insoluble polymer. Such polymer include films of polyolefins such as low density polyethylene, high density, polyethylene and polypropylene and films of elastomers such as ethylene-vinyl acetate copolymers, silicone elastomers natural rubber and polyurethane elastomers. Preferred polymers include high density polyethylene and polyurethane elastomers such as thermoplastic polyurethanes known as "Estanes" available from B.F. Goodrich & Co. Preferred "Estane" polyurethanes are grades 5714F and 58201.

Suitable perforate films will have a thickness of 12.5 to 100 micro metres and preferably a thickness of 25 to 50 micro metres.

The perforations in the perforate film suitable for

12

flexible tubes used in the invention should be capable of allowing the passage of body exudates to the absorbent tampon. The perforate films suitably have perforations of 1 to 55 mm$^2$ in area distributed at $25 \times 10^4$ to $1 \times 10$

$^4$ perforations per m$^2$ and preferably perforations of 4 to 25 mm$^2$ in area distributed at $9 \times 10^4$ to $2 \times 10^4$ perforations per m$^2$.

Favoured perforate films for the flexible tubes used in the invention include 25 to 50 micro metres thick films of high density polyethylene or polyurethane having performation 4 to 25 mm$^2$ in area distributed at $9 \times 10^4$ to $2 \times 10^4$ perforations per m$^2$.

The remaining part of the flexible tube which when extended does not surround the inserted tampon need not be liquid permeable. The remaining part of the flexible tube can comprise a tube structure made of a liquid impermeable flexible polymer film or polymer coated fabric. Suitable polymer films, fabrics and polymers for use in the liquid impermeable part of the flexible tube are described hereinbefore in relation to the liquid permeable flexible tubes. The liquid impermeable part of the flexible tube can be joined to liquid permeable part of the tube by any convenient method for example by adhesive or by heat

13

sealing.

The flexible tube can advantageously be attached at its collapsed portion end to or about the nose of the tampon. Such an attachment will cause the flexible tube sleeve to invert and roll away from walls of the body cavity when the tampon is withdrawn thus allowing the tampon to be withdrawn in a relatively comfortable manner. The flexible tube can also be attached to the rear end of the tampon which attachment will cause the flexible tube adjacent thereto to evert when the tampon is withdrawn. The flexible tube can be attached to the nose or rear end of the tampon by any suitable adhesive or mechanical means.

The flexible tube used in the invention can have a coating of a pharmaceutically acceptable lubricant or release agent as hereinbefore described to reduce friction between the tampon or the tampon cover and the flexible tube during insertion and to assist the unfolding of the collapsed portion.

The collapsed portion of the flexible tube used in the invention as hereinbefore mentioned will usually be in a folded configuration. The walls of collapsed portion can suitable have an axially extending series of circumferential folds which alternate in direction in for example a "zig-zag" pattern. Such folds can be obtained by compressing a portion of tube in direction parallel to the axis of the tube. Favoured flexible tubes for use in the invention therefore have a collapsed portion which comprises circumferential folds formed by axial compression of the tube. Apt flexible tubes have a folded collapsed portion in the form of compressed "bellows". A folded collapsed portion of the "bellows" type can be held in a compressed state by any suitable means for example adhesive means, physical means or mechanical means. The adhesive means include the use of water soluble adhesives. Physical means include a heat set treatment. Mechanical means can include an optionally water soluble wrapping film.

The length of the compressed axial "bellows" collapsed portion of the flexible tube is favourably less than 15 mm and for example 2 to 12 mm in length and preferably 5 to 10 mm in length. The compressed "bellows" configuration thus offers the advantage of compact storage of the collapsed portion of the flexible tube before use.

The collapsed portion of the flexible tube used in the invention unfolds during insertion of the tampon and

15

allows the cover portion of the tube to extend to form a sleeve about the tampon. Suitably the collapsed portion of the tube in its extended unfolded state can be at least 60 per cent of the length of the flexible tube, desirably at least 80 per cent and preferably at least 90 per cent of the length of the flexible tube. The collapsed portion in its extended unfolded state will surround the tampon after it has been inserted into the body cavity. It is therefore desirable that collapsed portion of the flexible tube comprises a liquid permeable material as hereinbefore described.

The collapsed portion of the flexible tube will usually be held in position at or about the nose of the tampon. Suitable means for holding the collapsed portion in position are described hereinbefore to the "bellows" configuration. In a preferred arrangment the collapsed portion of the tube is held in position by a cap which is attached to or about the nose of the tampon. Such a cap can provide a protective cover for the collapsed portion during insertion and in a preferred form can also provide the nose of the tampon with a smooth rounded shape to facilitate insertion of the device into a body cavity. The cap is preferably attached by for example adhesive or mechanical means to the centre portion of the nose of the

16

tampon to allow the collapsed portion of the flexible tube to unfold and to extend the cover portion over the peripheral surface of the tampon.

Suitable caps for holding the collapsed portion in position can comprise a plastic film which is preferably flexible film for example a polyolefin film such as a polyethylene film, a plastic coated paper or a plastics moulding. A preferred cap comprises a tube of polyolefin film fixed to the centre portion of the nose of a tampon and everted over the collapsed portion of the tube. The cap can, if convenient, be the front end portion of the flexible tube everted over the collapsed portion of the tube.

The cover portion of the flexible tube used in the invention covers at least a part of the tampon rearward of the nose thereof and is adjacent to the collapsed portion. The cover portion normally needs to be sufficiently long to provide good contact with the body cavity so that it extends from the collapsed portion when the tampon device is inserted into a body cavity. Suitably the cover portion of the tube can have a length which is at least one third of, desirably at least one half of and preferably can have a length which is at least the length of tampon rearward of the nose portion. The cover portion of the tube can have a length which is greater than the length of the tampon and which if desired can be attached to the rear end of the tampon.

The materials used for the tampon devices, tampon assemblies and applicators of invention are preferably biocompatible materials.

It is also preferred that the tampon devices, tampon assemblies and applicators of the invention are sterile to and preventing infection of the body cavity during insertion of the tampon. The tampon device, tampon assembly or applicator can be suitably packed into a bacteria-proof package and sterilised within the package by conventional methods.

The invention will be illustrated by reference to the following drawings:

Figure 1a shows a longitudinal section of a tampon device of the invention prior to insertion into vagina.

Figure 1b shows the tampon device of Figure 1a partially inserted into the vagina.

Figure 1c shows the tampon device of Figure 1a fully inserted in the vagina.

18

Figure 2 shows a longitudinal section of a second tampon device according to the invention.

Figure 3 shows a longitudinal section of a third tampon device according to the invention.

Figure 4 shows a longitudinal section of a fourth tampon device according to the invention.

Figure 5 shows a longitudinal section of a tampon assembly according to the invention.

Figure 6 shows a longitudinal section of an applicator according to the invention.

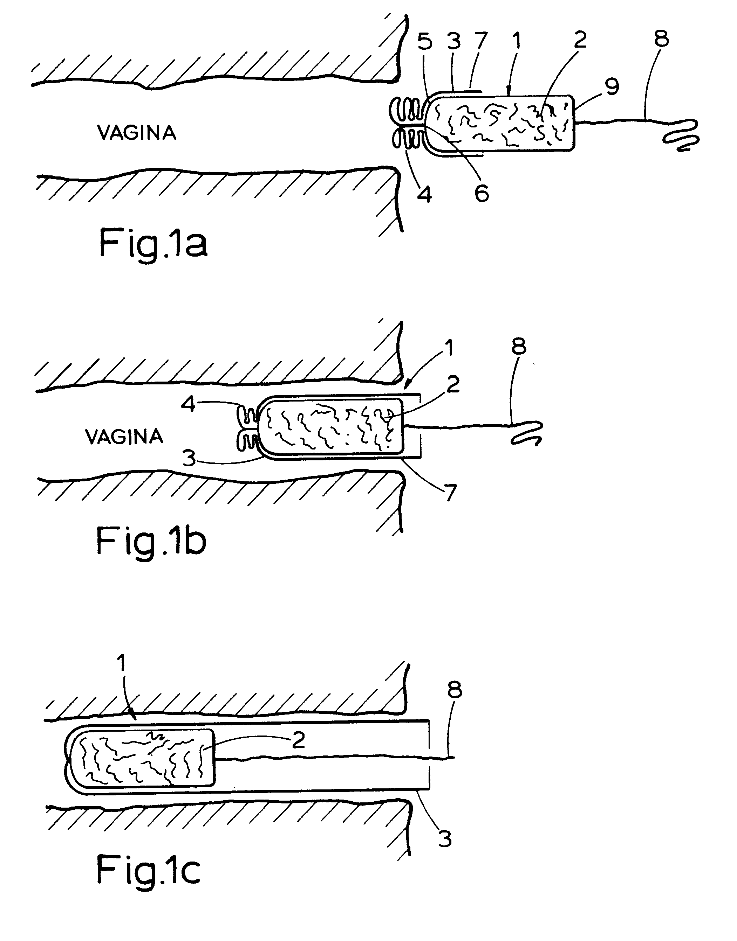

Figure 1a shows a tampon device 1 of the invention which comprises a tampon 2 and liquid permeable flexible tube 3 which has a collapsed portion 4 in the form of axially compressed folded "bellows" which is positioned at the nose 5 of the tampon and attached thereto at position 6. Adjacent to the collapsed portion 4 is a portion of the tube 7 which covers a part of tampon 2 rearward of the nose 5. The tampon device 1 is provided with a withdrawal string 8 which is attached at rear end 9 of the tampon.

The tampon device is shown in a position ready for

19

digital insertion into the opening of a vagina (shown diagramatically). Figure 1b shows the tampon device 1 partially inserted into the vagina. The cover portion 7 of the flexible tube 3 has extended from the collapsed portion 4 which is shown partially unfolded to form a sleeve around the moving tampon 2. Figure 1c shows the tampon device 1 fully inserted into the vagina and flexible tube 3 fully extended as a sleeve around the tampon 2. The unfolding action of the collapsed portion 4 enables the flexible tubular sleeve 3 to be layed down against the walls of the vagina in a substantially non frictional manner. Withdrawal of the tampon 2 from the vagina can be achieved by means of string 8. On withdrawal the flexible tubular sleeve 3 inverts with a rolling action which enables the tampon 2 to be withdrawn from the vagina in a non-frictional and therefore relatively comfortable manner.

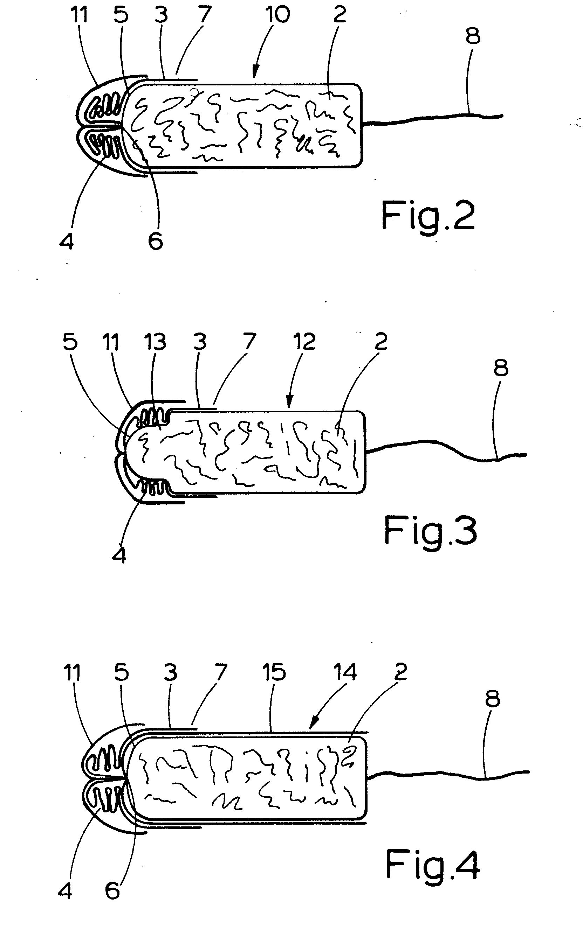

Figure 2 shows a tampon device 10 similar to that of Figure 1a except that the device has a protective cap 11 which covers the "bellows" collapsed portion 4 of the tube 3 and which is attached to the nose 5 at position 6. Cap 11 provides protection for the collapsed portion 4 during insertion and gives a rounded shape to the nose of the tampon to facilitate its insertion into the vagina.

20

Figure 3 shows tampon device 12 similar to that of Figure 2 except that the nose portion 13 of the tampon has a diameter smaller than that of the remaining portion of the tampon to accomodate the axially compressed bellows 4 portion of the tube 3 positioned about the nose 5 of the tampon.

Figure 4 shows a tampon device 14 similar to that shown in Figure 2 with an inner shroud 15 which surrounds the tampon 2 to reduce friction between the extending cover portion 7 of the flexible tube and the tampon 2 when the device 14 is inserted into the vagina.

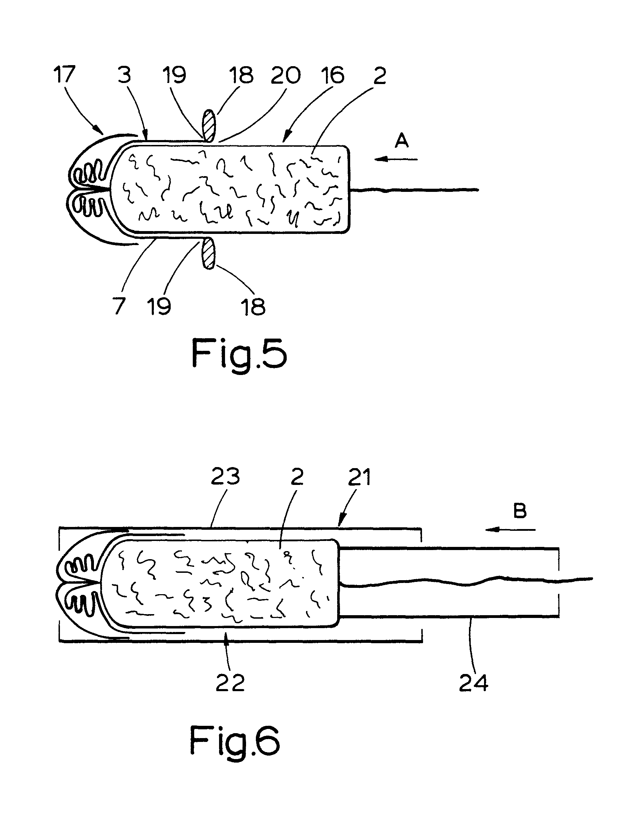

Figure 5 shows a tampon assembly 16 for aiding the insertion of tampon 4 consisting of tampon device 17 which is similar to that of Figure 2 and a tampon guide 18 which is attached at position 19 to cover portion 7 of the flexible tube 3. The tampon guide 18 has an opening 20 which can be placed over the body cavity opening during insertion of the tampon therein.

The tampon device 17 can be inserted through the opening 20 in the tampon guide 18 by means of digital pressure applied in direction of arrow A. Preferably the attachment at position 19 is frangible so that the guide

21

can be separated from the cover portion 7 of the flexible tube when the tampon is inserted into the body cavity.

Figure 6 shows an applicator 21 for aiding insertion of tampon 2 which consists of a tampon device 22 similar to that of Figure 2 and applicator tube 23 containing the tampon device 22 and a piston tube 24 capable of sweeping the applicator tube 23. The tampon device 22 can be ejected from applicator tube 23 placed adjacent to a body cavity opening (not shown) by movement of the piston tube 24 in the direction shown by arrow B.

- 1 -

CLAIMS

1.    A tampon device for insertion into a body cavity which device comprises a tampon and a flexible tube adapted to allow transitional movement of the tampon therein, which tube has a collapsed portion at or about the nose of the tampon and a portion adjacent to the collapsed portion which covers at least part of the tampon rearward of the nose thereof and which portion is capable of extending from the collapsed portion to form a sleeve about the tampon when the device is being inserted into a body cavity.

2.    A tampon device as claimed in claim 1 in which the flexible tube is attached to or about the nose of the tampon.

3.    A tampon device as claimed in either of claims 1 or 2 in which the collapsed portion of the flexible tube comprises circumferential folds formed by axial compression of the tube.

4.    A tampon device as claimed in any of claims 1 to 3 in which the tampon has a nose portion which has a

- 2 -

diameter which is smaller than that of the main body of the tampon to accommodate a collapsed portion of the flexible tube positioned about the nose of the tampon.

5.    A tampon device as claimed in any of claims 1 to 4 in which the collapsed portion of the flexible tube is held in position by a cap which is attached to the nose of the tampon.

6.    A tampon device as claimed in claim 5 in which the cap comprises a flexible plastics film.

7.    A tampon device as claimed in any of claims 1 to 6 in which the flexible tube comprises a liquid permeable non-woven fabric.

8.    A tampon device as claimed in any of claims 1 to 7 in which the tampon has a cover layer of liquid permeable material which surrounds the tampon to reduce friction between the surface of the tampon and the flexible tube during insertion of the tampon into a body cavity.

9.    A tampon assembly for aiding the insertion of a tampon into a body cavity comprising a tampon device which comprises a tampon and a flexible tube, adapted to allow transitional movement of the tampon therein, which tube has a collapsed portion at or about the nose of the tampon and a portion adjacent to the collapsed portion which covers at least a part of the tampon rearward of the nose thereof and which portion is capable of extending from the collapsed portion to form a sleeve about the moving tampon when the device is being inserted into a body cavity and a tampon guide for receiving the tampon device having an opening to be placed over the body cavity opening.

10.    An applicator for aiding the insertion of a tampon into a body cavity comprising (1) a tampon device which comprises a tampon and a flexible tube, adapted to allow transitional movement of the tampon therein, which tube has a collapsed portion at or about the nose of the tampon and a portion adjacent to the collapsed portion which covers at least a part of the tampon rearward of the nose thereof and which portion is capable of extending from the collapsed portion to form a sleeve about the moving tampon when the device is being inserted into the body cavity;  (2) an applicator tube containing the tampon device and (3) a piston capable of sweeping the applicator tube and ejecting the tampon device therefrom.

Fig.1a

Fig.1b

Fig.1c

Fig.2

Fig.3

Fig.4

Fig.5

Fig.6